# EUROPEAN PATENT SPECIFICATION

(11) **EP 0 411 035 B1**
(45) Date of publication and mention of the grant of the patent: **30.11.1994**
(21) Application number: 89905340.9
(22) Date of filing: 29.03.1989
(51) Int. Cl.: C07C 67/08, C07C 69/63

(54) **PROCESS FOR PREPARING 2-BROMOETHYL ACETATE**
VERFAHREN ZUR HERSTELLUNG VON 2-BROMOETHYLACETAT
PROCEDE POUR PREPARER DU 2-BROMOETHYL-ACETATE

(30) Priority: 11.04.1988 US 179848
(43) Date of publication of application: 06.02.1991
(73) Proprietor: MALLINCKRODT, INC., St. Louis, MO 63134 (US)
(72) Inventor: CHAPMAN, Robert, C., St. Louis, MO 63134 (US)
(74) Representative: Allard, Susan Joyce
(86) International application number: US8901298
(87) International publication number: WO8909763

(56) References cited:
- FR-A- 1 334 467
- US-A- 4 314 071
- JOURNAL OF THE CHEMICAL SOCIETY, Perkin Transactions I, 1973, London, GB; B. T. GOLDING et al, "Reaction between vicinal diols and hydrogen bromide in acetic acid; synthesis of chiral propylene oxide", pages 1214-1220
- BEILSTEINS HANDBUCH DER ORGANISCHEN CHEMIE, Drittes Ergänzungswerk, 4th edition, vol. 2, part 1, 1960, Springer-Verlag, Berlin, DE, page 227

## Description

This invention relates to the preparation of 2-bromoethyl acetate and, more particularly, to an improved process for the preparation of 2-bromoethyl acetate which is more cost effective and which produces this compound in good to excellent yield with excellent purity.

2-Bromoethyl acetate is a compound which is employed in the preparation of 5-[N-(2-acetoxyethyl) acetoxyacetamido]-N,N'-bis(2,3-diacetoxypropyl)-2,4,6-triiodoisophthalamide which in turn is an intermediate in the preparation of N,N'-bis(2,3-dihydroxypropyl)-5-[N-(2-hydroxyethyl)glycolamido]-2,4,6-triiodoisophthalamide. The latter compound is a nonionic x-ray contrast agent (see Lin U.S. patent no. 4,396,598 dated August 2, 1983).

Heretofore, it has been known to prepare 2-bromoalkyl acetates (bromohydrin acetates) from 1,2-diols such as ethylene glycol by reacting the diol with 6 molar hydrogen bromide in acetic acid. Golding et al., J. Chem. Soc. Perkin Trans. I, 1973,1214, Bhat et al. Syn. 142 (1984) and Blomquist et al., J. Am. Chem. Soc. 74, 3636 (1952). This preparation involved the use of anhydrous gaseous hydrogen bromide or a saturated solution of hydrogen bromide in acetic acid and requires removal of the water produced by the reaction.

There has been a need in the art for an improved process for preparing 2-bromoethyl acetate which is more cost effective and produces a product of excellent purity in high yield.

### Summary of the Invention

Among the several objects of the present invention, therefore, may be noted the provision of an improved process of 2-bromoethyl acetate; the provision of such a process which is more cost effective and produces a product of good quality; and the provision of such a process which is readily carried out and which facilitates the preparation of a key intermediate in the production of a nonionic x-ray contrast agent. Other objects and features will be in part apparent and in part pointed out hereinafter.

Briefly, the present invention is directed to an improvement in a process for preparing 2-bromoethyl acetate, the improvement comprising reacting ethylene glycol with an aqueous solution of hydrogen bromide and acetic acid under reflux conditions at atmospheric pressure in the presence of a solvent such as toluene which forms an azeotrope with water but not with 2-bromoethyl acetate and distilling off an azeotrope containing water and the solvent wherein the reactants ethylene glycol and hydrogen bromide are present in substantially equimolar equivalents. The process of the present invention may also include the feature of heating the reaction mixture to remove the azeotrope and adding acetic anhydride or acetic acid to the reaction mixture to convert bromoethanol in the mixture to additional 2-bromoethyl acetate product. A further feature of the process may also include the addition of an antioxidant and a base with the acetic anhydride or acetic acid added to the reaction mixture before distillation to prevent discoloration and to take up any excess free hydrogen bromide.

### Description of the Preferred Embodiments

In accordance with the present invention, it has now been found that 2-bromoethyl acetate may be prepared in high yield, good quality and less costly by reacting ethylene glycol with an aqueous solution of hydrogen bromide and acetic acid under reflux conditions at atmospheric pressure in the presence of a solvent which forms an azeotrope with water but not with 2-bromoethyl acetate and distilling off an azeotrope containing water and the solvent wherein the reactants ethylene glycol and hydrogen bromide are present in substantially equimolar equivalents. Considerable cost savings are realized by utilizing an aqueous solution of hydrogen bromide such as a 48% aqueous solution rather than employing anhydrous gaseous hydrogen bromide as has been done in the past. Moreover, it has been found that carrying out the reaction in the presence of a solvent which forms an azeotrope with water but not with 2-bromoethyl acetate greatly facilitates the removal of the water normally generated by the reaction enabling a greater yield of the desired 2-bromoethyl acetate to be realized.

In the practice of the invention, ethylene glycol, acetic acid, an aqueous solution of hydrogen bromide and a solvent such as toluene are combined into a reaction mixture and the reaction mixture is heated under reflux conditions at atmospheric pressure to distill an azeotrope containing water, the solvent and some unreacted acetic acid. Surprisingly, there appears to be substantially no loss of hydrogen bromide from the reaction mixture under these conditions. The distillate obtained separates into a water layer and a solvent layer, both of which contain acetic acid. The process is operated continuously with the solvent layer being continuously fed back or recycled to the reaction mixture and the water formed in the reaction which produces 2-bromoethyl acetate being continuously removed as an azeotrope with the solvent. When the water has been substantially removed from the reaction mixture, the latter contains approximately 90% 2-bromoethyl acetate and approximately 10% bromoethanol which is formed by reaction between hydrogen bromide and ethylene glycol.

In order to convert the bromoethanol into the desired 2-bromoethyl acetate, either acetic acid or acetic anhydride are added to the reaction mixture in an amount necessary to react with all bromoethanol present in the mixture. The use of acetic anhydride is preferred since it performs a dual function, i.e. it reacts with any water present to form acetic acid which in turn reacts with bromoethanol to produce additional 2-bromoethyl acetate. After the addition of acetic acid or acetic anhydride, the reaction mixture is subjected to further heating and distillation of the mixture is continued until all of the azeotrope containing water and the solvent together with excess acetic acid has been removed from the mixture. At this point, essentially all of the reactants including any bromoethanol have been converted to the desired 2-bromoethyl acetate product.

As indicated, an important feature of the improved process of the invention resides in carrying out the reaction in the presence of a solvent which forms an azeotrope with water but not with 2-bromoethyl acetate. Toluene is the preferred solvent of this type because of its ready availability. In lieu of toluene, other solvents which form such an azeotrope include 1,1,2-trichloroethane, 1,1,1-trichloroethane, benzene and methylene chloride, but those skilled in the art will appreciate that still other solvents forming the desired azeotrope may also be employed.

In another aspect of the invention, it has been found advantageous to add an antioxidant and a base to the reaction mixture along with the acetic acid or acetic anhydride added to convert bromoethanol to additional 2-bromoethyl acetate. These materials prevent discloration of the reaction mixture and the base also acts as an acid scavenger to take up any free excess hydrogen bromide. The preferred antioxidant for use in the practice of the invention is sodium bisulfite, but other antioxidants known to the art such as nitric oxide and benzoquinone may also be employed. The preferred base is sodium carbonate, but any alkali metal carbonate or bicarbonate or comparable weak base may also be used.

An illustrative specific embodiment of the process of the invention may be described as follows. The bottom valve of a clean and dry 113.56ℓ (30 gallon) reactor is closed. Using a vacuum acid/organic respirator, 12.9 kg of acetic acid are transferred to the reactor from a nitrogen flushed, grounded drum. Next 18 kg of toluene is vacuum transferred to the reactor from a grounded drum in a hood and 35 kg of a 48% aqueous solution of hydrogen bromide are slowly vacuum transferred from the hood to the reactor. The reaction mixture is then distilled to a receiver adapted to reflux the upper toluene layer while separating and removing the lower water layer until 31.42ℓ (8.3 gallons) of water have been collected. Five additional kg of acetic acid is added to the reaction mixture by gravity and distillation is continued for two more hours. The system is then changed to total take off on the distillation and the reaction mixture is distilled until all toluene and acetic acid have been removed. The toluene/acetic acid in the receiver is then drained into a grounded, nitrogen flushed drum and used in a subsequent run. The 2-bromoethyl acetate product is then distilled under reduced pressure (boiling point approximately 80°C at 70 mm).

In lieu of adding the acetic acid in the above described procedure, the reaction mixture is cooled to 20-30°C after the initial distillation step and approximately 2.6 kg of acetic anhydride or other amount as needed based on the amount of bromoethanol plus water remaining in the reaction mixture is slowly transferred by vacuum with stirring from a grounded, nitrogen flushed drum to the reactor. The reaction mixture is stirred for 2 hours at 25-35°C, the reaction mixture is resampled and additional acetic anydride as needed is added to react with all bromoethanol present in the reaction mixture. 92 g of sodium bisulfite (NaHSO₃) is then charged to the reactor along with an amount of sodium carbonate equal to 92 g plus an amount based on the strong acid value of the reaction mixture. The process then proceeds as described above.

The improved process of the invention thus offers significant advantages for preparing 2-bromoethyl acetate in a practical, cost effective and convenient manner and in good yield and purity.

The following examples illustrate the practice of the invention.

### Example 1

To a suspension of ethylene glycol (6.2 g, 0.1 mol), acetic acid (6.3 ml, 0.11 mol) and toluene (30 ml) was added a 48% aqueous solution of hydrogen bromide (11.8 ml, 0.105 mol), and the mixture was refluxed under Dean Stark conditions for 6 hr. After 4 hr., approximately 11 ml. of water had been collected and after 6 hr., 12.8 ml. of water had been collected. The distillate was removed and distillation was continued at a temperature of approximately 116°C until all toluene and unreacted acetic acid had been removed as evidenced by gas chromatography. The product mixture contained 92 area % 2-bromoethyl acetate and 8 area % bromoethanol.

### Example 2

To a solution of ethylene glycol (56 ml, 1.0 mol) and acetic acid (74 ml, 1.3 mol) in toluene (100 ml) was added a 48% aqueous solution of hydrogen bromide (118 ml, 1.05 mol) and the mixture was refluxed under Dean Stark conditions until 125 ml of water had been collected. At this point, gas chromatography showed that the reaction mixture contained approximately 10% bromoethanol. Additional acetic acid (11.4 ml, 0.2 mol) was added and refluxing was continued for 2 hr. at which time the reaction mixture contained approximately 96.5% 2-bromoethyl acetate and 3.5% bromoethanol. Additional acetic acid (11.4 ml, 0.2 mol) was added and refluxing continued for an additional 2 hr. at which time the reaction mixture contained approximately 3% bromoethanol. After stirring the mixture overnight, approximately 2.4 area % bromoethanol remained.

### Example 3

A mixture of ethylene glycol (56 ml, 1.0 mol), acetic acid (74.4 ml, 1.3 mol), a 48% aqueous solution of hydrogen bromide (112 ml, 1.0 mol) and toluene (100 ml) was heated under reflux under Dean Stark conditions until 128 ml of water had been collected. The water which separated from the distillate contained 13 wt% of acetic acid. More acetic acid (11.4 ml, 0.2 mol) was added and the refluxing continued at a temperature of 106°C. Another portion of acetic acid (11.4 ml, 0.2 mol) was added. A total of approximately 133 ml of water was collected. The material was distilled under 20 mm pressure to remove excess acetic acid and toluene. The pot residue after distilling at head temperatures up to 62°C at 20 mm contained 122.6g (73.4% yield) of 2-bromoethyl acetate which contained 4 area % bromoethanol.

### Example 4

To a 4-necked flask were added ethylene glycol (279 ml, 310 g, 5 mol), acetic acid (372 ml, 290 g, 6.5 mol), toluene (500 ml) and an aqueous 48% solution of hydrogen bromide (562 ml, 5 mol) with stirring. The mixture was refluxed under Dean Stark conditions until 665 ml of a water layer had been collected. To this mixture was added acetic anhydride (23.5 ml, 0.25 mol) and the mixture was heated for approximately 25 more minutes. Some bromine evolution was noted. With the reaction mixture at 120°, there was some darkening of the solution. Gas chromatography showed that the mixture contained approximately 4.5% bromoethanol. The reaction mixture was cooled and additional acetic anhydride (23.5 ml, 0.25 mol) was added. After additional heating, gas chromatography showed that the mixture now contained approximately 2.8% bromoethanol.

### Example 5

To a 3 liter, 4-necked flask fitted with a mechanical stirrer was gradually added ethylene glycol (279 ml, 5 mol), acetic acid (372 ml, 6.5 mol), toluene (400 ml) and an aqueous 48% hydrogen bromide solution (562 mol, 5 mol). The mixture was refluxed under Dean Stark conditions until 750 ml. of a bottom layer of distillate containing water was collected. The mixture was cooled to 25-30°. Gas chromatography showed 14% bromoethanol. The reaction mixture also contained 8.9 g water (0.5 mol). Enough acetic anhydride (113 ml, 1.2 mol) was added to react with residual water and bromoethanol. During the addition, the temperature rose to approximately 50°C, then cooled to 30°C. Gas chromatography showed that the mixture contained less than 0.2% bromoethanol. The material was divided into aliquots.

The solvent was distilled from one aliquot after the addition of 0.1 g of sodium bisulfite to the mixture. The distillate was colorless.

Upon distillation of another aliquot with nothing added, the material darkened.

To the crude mixture (50 ml, 60 g) produced above before dividing into aliquots was added 0.1 g sodium bisulfite and 0.18 g sodium carbonate and the mixture was distilled at 100 mm pressure until the head temperature stabilized. Very little color formation was noted.

### Example 6

To a 113.56ℓ (30 gal) reactor was added ethylene glycol (12.9 kg, 208 mol) followed by glacial acetic acid (16.2 kg, 270 mol). The agitator was started and toluene (33 kg) was added. To this mixture at 20°C was charged an aqueous 48% hydrogen bromide solution (35 kg, 208 mol) and the reaction mixture was heated under reflux conditions for about 2 hr followed by partial reflux conditions from a receiver adapted to reflux the upper toluene layer while allowing collection of the lower water layer. This procedure was continued until 29.34ℓ (7 and 3/4 gallons) of the lower layer had been collected. The reaction mixture was sampled and the water and bromoethanol levels were measured. The batch was determined to contain 28 mol water and 41 mol bromoethanol. The reaction mixture was cooled to room temperature and acetic anhydride (7.0 kg, 69 mol) was added slowly with stirring and water cooling. The mixture was allowed to stand overnight at room temperature. The excess acetic acid and toluene were then removed by vacuum distillation at 25 mm to a maximum pot temperature of 76°C. The material in the pot was cooled and filtered through a 10 micron filter giving 32.2 kg of 2-bromoethyl acetate. Assay = 95.5%; yield = 92.8%.

### Example 7

To a 113.56ℓ (30 gal) reactor was charged ethylene glycol (12.9 kg, 208 mol) followed by addition with stirring of glacial acetic acid (16.3 kg, 271 mol), toluene (25.5 kg) and an aqueous 48% hydrogen bromide solution (35.0 kg, 208 mol). The mixture was heated and distilled with partial reflux of the upper toluene layer as described above until approximately 28.39ℓ (7.5 gal) water had been collected and the pot temperature reached 119°C. The mixture was cooled and a sample was analyzed for bromoethanol (25 moles) and water (17 moles) content. Acetic anhydride (4.3 kg, 42 moles) was added slowly to the reaction mixture and it was stirred an additional hour at 28°C. To the reaction mixture were added sodium bisulfite (92 g) and sodium carbonate (141 g) and the mixture was allowed to stand overnight at ambient temperature. The excess toluene and acetic acid were removed under reduced pressure (29.5 in, pot temp. to 84°C). The 2-bromoethyl acetate which remained in the pot was cooled to room temperature and filtered through a 10 micron filter yielding 33.2 kg product. Yield = 95.7%; Assay = 101%.

In view of the above, it will be seen that the several objects of the invention are achieved and other advantageous results attained.

## Claims

1. A process for preparing 2-bromoethyl acetate by reacting ethylene glycol, hydrogen bromide and acetic acid, characterised by reacting under reflux conditions at atmospheric pressure ethylene glycol with an aqueous solution of hydrogen bromide and acetic acid in the presence of a solvent which forms an azeotrope with water but not with 2-bromoethyl acetate and distilling off an azeotrope containing water and the solvent, wherein the reactants ethylene glycol and hydrogen bromide are present in substantially molar equivalents.

2. A process as claimed in claim 1 wherein said solvent is selected from toluene, 1,1,2-trichloroethane, 1,1,1-trichloroethane, benzene or methylene chloride.

3. A process as claimed in claim 1 wherein said solvent is toluene.

4. A process as claimed in any one of claims 1 to 3 wherein said aqueous solution of hydrogen bromide is a 48% aqueous solution.

5. A process as claimed in any one of claims 1 to 4 further comprising removing water from the reaction mixture containing said 2-bromoethyl acetate.

6. A process as claimed in claim 5 wherein the reaction mixture is heated to remove said solvent and water therefrom.

7. A process as claimed in claim 6 wherein a compound selected from acetic anhydride or acetic acid is subsequently added to the reaction mixture to convert bromoethanol in said mixture to additional 2-bromoethyl acetate and remove residual water.

8. A process as claimed in claim 7 wherein an antioxidant selected from sodium bisulfite, nitric oxide or benzoquinone and a base selected from alkali metal carbonates or bicarbonates are added to said reaction mixture with said compound selected from acetic anhydride or acetic acid.

9. A process as claimed in claim 8 wherein said compound is acetic anhydride, said antioxidant is sodium bisulfite and said base is sodium carbonate.

10. A process for preparing 2-bromoethyl acetate as claimed in claim 1 which comprises reacting ethylene glycol with an aqueous solution of hydrogen bromide and acetic acid in the presence of toluene, the reactants being present in substantially molar equivalents, heating the resulting reaction mixture under reflux conditions at atmospheric pressure to separate toluene and water therefrom, adding acetic anhydride to the reaction mixture to convert bromoethanol therein to additional 2-bromoethyl acetate, and distilling the resulting mixture to remove toluene, water and any remaining acetic acid therefrom thereby producing 2-bromoethyl acetate.

## Patentansprüche

1. Verfahren zum Herstellen von 2-Bromethylacetat durch Umsetzen von Ethylenglykol, Bromwasserstoff und Essigsäure, gekennzeichnet durch Umsetzen von Ethylenglykol unter Rückflußbedingungen bei atmosphärischem Druck mit einer wäßrigen Lösung von Bromwasserstoff und Essigsäure in der Anwesenheit eines Lösungsmittels, das ein Azeotrop mit Wasser aber nicht mit 2-Bromethylacetat bildet, und Abdestillieren eines Azeotrops enthaltend Wasser und das Lösungsmittel, worin die Reaktanden Ethylenglykol und Bromwasserstoff im wesentlichen in molarer Äquivalenz vorliegen.

2. Verfahren wie in Anspruch 1 beansprucht, worin das Lösungsmittel ausgewählt wird aus Toluol, 1,1,2-Trichlorethan, 1,1,1-Trichlorethan, Benzol oder Methylenchlorid.

3. Verfahren wie in Anspruch 1 beansprucht, worin das Lösungsmittel Toluol ist.

4. Verfahren wie in einem der Ansprüche 1 bis 3 beansprucht, worin die wäßrige Lösung aus Bromwasserstoff eine 48%-ige wäßrige Lösung ist.

5. Verfahren wie in einem der Ansprüche 1 bis 4 beansprucht, das weiterhin umfaßt das Entfernen von Wasser aus der Reaktionsmischung enthaltend das 2-Bromethylacetat.

6. Verfahren wie in Anspruch 5 beansprucht, worin die Reaktionsmischung erwärmt wird, um das Lösungsmittel und Wasser daraus zu entfernen.

7. Verfahren wie in Anspruch 6 beansprucht, worin eine Verbindung ausgewählt aus Essigsäureanhydrid oder Essigsäure, anschließend der Reaktionsmischung zugesetzt wird, um Bromethanol in der Mischung zu weiterem 2-Bromethylacetat umzuwandeln und restliches Wasser zu entfernen.

8. Verfahren wie in Anspruch 7 beansprucht, worin ein Antioxidans, ausgewählt aus Natriumbisulfit, Stickoxid oder Benzochinon und eine Base, ausgewählt aus Alkalimetallcarbonaten oder Bicarbonaten, zu der Reaktionsmischung mit der Verbindung, ausgewählt aus Essigsäureanhydrid oder Essigsäure, zugesetzt werden.

9. Verfahren wie in Anspruch 8 beansprucht, worin die Verbindung Essigsäureanhydrid ist, das Antioxidans Natriumbisulfit ist und die Base Natriumcarbonat ist.

10. Verfahren zum Herstellen von 2-Bromethylacetat wie in Anspruch 1 beansprucht, das umfaßt Umsetzen von Ethylenglykol mit einer wäßrigen Lösung von Bromwasserstoff und Essigsäure in der Anwesenheit von Toluol, wobei die Reaktanden im wesentlichen in molarer Äquivalenz vorliegen, Erwärmen der erhaltenen Reaktionsmischung unter Rückflußbedingungen bei atmosphärischem Druck, um Toluol und Wasser davon abzutrennen, Zusetzen von Essigsäureanhydrid zu der Reaktionsmischung, um darin enthaltenes Bromethanol zu weiterem 2-Bromethylacetat umzuwandeln, und Destillieren der erhaltenen Mischung, um Toluol, Wasser und sämtliche verbliebene Essigsäure daraus zu entfernen, und dadurch Herstellen von 2-Bromethylacetat.

## Revendications

1. Procédé pour préparer de l'acétate de 2-bromoéthyle par la réaction de l'éthylèneglycol, du bromure d'hydrogène et de l'acide acétique, procédé caractérisé en ce qu'on fait réagir, dans des conditions de reflux sous la pression atmosphérique, de l'éthylèneglycol avec une solution aqueuse de bromure d'hydrogène (acide bromhydrique) et d'acide acétique en présence d'un solvant formant un azéotrope avec l'eau mais non pas avec l'acétate de 2-bromoéthyle et en ce qu'on chasse par distillation un azéotrope contenant l'eau et le solvant, l'éthylène glycol et le bromure d'hydrogène que l'on met en réaction étant présents en des quantités d'équivalents sensiblement molaires.

2. Procédé tel que revendiqué à la revendication 1, dans lequel ledit solvant est choisi parmi le toluène, le 1,1,2-trichloréthane, le 1,1,1-trichloroéthane, le benzène ou le chlorure de méthylène.

3. Procédé tel que revendiqué à la revendication 1, dans lequel ledit solvant est du toluène.

4. Procédé tel que revendiqué dans l'une quelconque des revendications 1 à 3, dans lequel ladite solution aqueuse de bromure d'hydrogène (acide bromhydrique) est une solution aqueuse à 48 %.

5. Procédé tel que revendiqué dans l'une quelconque des revendications 1 à 4, comprenant en outre l'enlèvement de l'eau du mélange réactionnel contenant ledit acétate de 2-bromoéthyle.

6. Procédé tel que revendiqué à la revendication 5, dans lequel on chauffe le mélange réactionnel pour en enlever ledit solvant et l'eau.

7. Procédé tel que revendiqué à la revendication 6, dans lequel on ajoute ensuite au mélange réactionnel un composé choisi parmi l'anhydride acétique ou l'acide acétique pour convertir le bromoéthanol présent dans le "mélange" en un supplément d'acétate de 2-bromoéthyle et enlever l'eau résiduelle.

8. Procédé tel que revendiqué à la revendication 7, dans lequel on ajoute un antioxydant, choisi parmi le bisulfite de sodium, l'oxyde nitrique ou la benzoquinone, et une base choisie parmi des carbonates ou bicarbonates de métaux alcalins, audit mélange réactionnel avec ledit composé choisi parmi l'anhydride acétique ou l'acide acétique.

9. Procédé tel que revendiqué à la revendication 8, dans lequel ledit composé est l'anhydride acétique, ledit antioxydant est le bisulfite de sodium et ladite base est le carbonate de sodium.

10. Procédé pour préparer de l'acétate de 2-bromoéthyle, tel que revendiqué à la revendication 1, qui comprend la réaction de l'éthylèneglycol avec une solution aqueuse de bromure d'hydrogène (acide bromhydrique) et d'acide acétique en présence de toluène, les corps devant réagir étant présents en des équivalents sensiblement molaires, le chauffage du mélange réactionnel résultant, dans des conditions de reflux sous la pression atmosphérique, pour séparer le toluène et l'eau du mélange réactionnel, l'addition d'anhydride acétique au mélange réactionnel pour convertir le bromoéthanol présent dans ce mélange en un supplément d'acétate de 2-bromoéthyle, et la distillation du mélange résultant pour enlever le toluène, l'eau et tout l'acide acétique éventuellement restant, en produisant ainsi de l'acétate de 2-bromoéthyle.
